(19) Europäisches Patentamt
European Patent Office
Office européen des brevets

(11) EP 3 406 589 B1

(12) **EUROPÄISCHE PATENTSCHRIFT**

(45) Veröffentlichungstag und Bekanntmachung des Hinweises auf die Patenterteilung:
**08.04.2020 Patentblatt 2020/15**

(51) Int Cl.:
*C07C 209/48* (2006.01) *C07C 209/52* (2006.01)
*C07C 253/30* (2006.01)

(21) Anmeldenummer: **17172376.0**

(22) Anmeldetag: **23.05.2017**

(54) **VERFAHREN ZUR HERSTELLUNG VON AMINOVERBINDUNGEN AUS NITRILVERBINDUNGEN**
METHOD FOR THE PRODUCTION OF AMINO COMPOUNDS FROM NITRILE COMPOUNDS
PROCÉDÉ DE FABRICATION DE COMPOSÉS AMINO À PARTIR DE COMPOSÉS NITRILE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**28.11.2018 Patentblatt 2018/48**

(73) Patentinhaber: **Evonik Operations GmbH**
**45128 Essen (DE)**

(72) Erfinder:
- **RITTSTEIGER, Anne**
  **4233 Meltingen (CH)**
- **KOHLSTRUK, Stephan**
  **45966 Gladbeck (DE)**
- **HOPPE, Dirk**
  **48301 Nottuln (DE)**
- **RÜFER, Alexander Martin**
  **46282 Dorsten (DE)**
- **SOWKA, Sabrina**
  **48249 Dülmen (DE)**
- **SCHNEIDER, Sven**
  **45711 Datteln (DE)**
- **SCHLÜTER, Norbert**
  **48712 Gescher (DE)**
- **HENGSTERMANN, Axel**
  **48308 Senden (DE)**
- **GALLE, Markus**
  **44357 Dortmund (DE)**
- **RÖDER, Stefan**
  **36391 Sinntal (DE)**
- **BERWEILER, Monika**
  **63477 Maintal (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(56) Entgegenhaltungen:
**WO-A1-2012/126869** **WO-A1-2012/126956**
**WO-A1-2016/030383**

EP 3 406 589 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein verbessertes Verfahren zur Herstellung von Aminoverbindungen aus Nitrilverbindungen. Nitrilgruppen können über Hydrierung in Aminomethylgruppen umgewandelt werden. Ggf. weitere reduzierbare funktionelle Gruppen, wie z.B. Iminogruppen, können dabei ebenfalls hydriert werden. In Gegenwart von Ammoniak können weiterhin insbesondere Ketonitrile über aminierende Hydrierung (ein- oder mehrstufige Umwandlung von Oxogruppen in Aminogruppen in Gegenwart von Ammoniak sowie Reduktion der Nitrilgruppen zu Aminomethylgruppen) in Aminoverbindungen (mit mindestens zwei Aminogruppen) umgewandelt werden. Insbesondere betrifft die vorliegende Erfindung ein verbessertes Verfahren zur aminierenden Hydrierung von 3-Cyano-3,5,5-trimethylcyclohexanon, auch Isophoronnitril oder abgekürzt IPN genannt, zu 3-Aminomethyl-3,5,5-trimethylcyclohexylamin, auch Isophorondiamin oder abgekürzt IPDA genannt.

Die Herstellung von IPDA durch aminierende Hydrierung von IPN ist bekannt und bereits mehrfach beschrieben worden.

Im einfachsten Fall (US 3,352,913 A) wird IPN in Gegenwart von Wasserstoff und einem Überschuss an Ammoniak an einem Cobaltkatalysator zur Reaktion gebracht. Zunächst bildet sich aus IPN und Ammoniak durch Wasserabspaltung das Isophoronnitrilimin, IPNI, das nachfolgend zum IPDA hydriert wird.

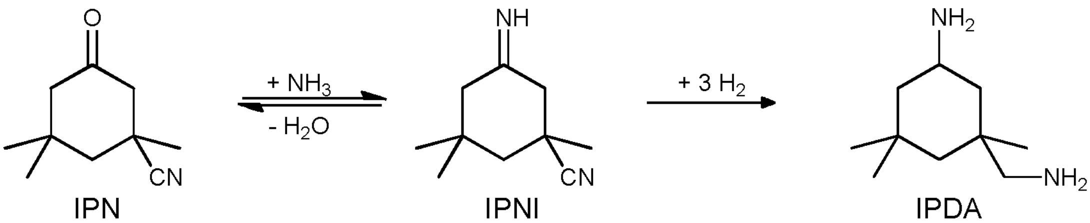


IPN IPNI IPDA

Die Ausbeute an IPDA wird bei dieser Art der Reaktionsführung maßgeblich durch den Überschuss an Ammoniak bestimmt. Die maximal erzielten IPDA-Ausbeuten liegen bei ca. 80 %. Hauptnebenprodukt ist der sogenannte Aminoalkohol, IPAA, der aus der direkten Hydrierung des IPN resultiert.

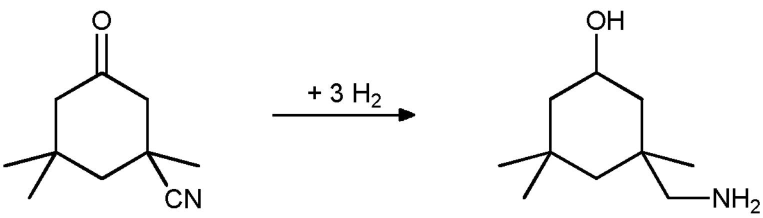


IPN IPAA

Eine wesentliche Steigerung der IPDA-Ausbeute wird erreicht, wenn die Bildung des IPNI durch Einsatz geeigneter Iminierungskatalysatoren beschleunigt wird. Als Iminierungskatalysatoren sind z.B. saure Ionenaustauscherharze (EP 0 042 119 B1) geeignet. Daneben können auch acide Metalloxide (EP 0 449 089 B1), sulfonsäuregruppenhaltige Organopolysiloxane (EP 0 816 323 A1), Heteropolysäuren (DE 44 26 472 A1) und Aktivkohle (EP 0 623 585 A1) als Iminierungskatalysatoren eingesetzt werden. Neben der Reduzierung des unerwünschten Aminoalkohols werden auch andere Nebenprodukte deutlich zurück gedrängt, z.B. bicyclische Verbindungen und solche Nebenprodukte, die aus der Abspaltung von HCN resultieren.

Auf die Problematik der Abspaltung von HCN aus gamma-Ketonitrilen, wie IPN, wird in der Literatur besonders hingewiesen (US 3,352,913 A). Zum einen wird bemerkt, dass durch die HCN-Abspaltung die Ausbeute an IPDA reduziert wird (EP 0 042 119 B1, DE 44 26 472 A1). Zum anderen wird darauf hingewiesen, dass HCN als Katalysatorgift wirkt und zu einer Desaktivierung des Hydrierkatalysators führt (EP 0 394 967 A1, Seite 2 Zeile 34 ff, Seite 3 Zeile 44 ff). Es wird daher empfohlen, den Iminierungsschritt so durchzuführen, dass möglichst kein HCN abgespalten wird.

Gemäß EP 0 913 387 B1 können zur Selektivitätssteigerung bei der Herstellung von IPDA auch quaternäre Ammoniumbasen eingesetzt werden. Entsprechend modifizierte Katalysatoren weisen speziell bei Verwendung eines Lösungsmittels eine deutlich höhere Standzeit auf als alkalimodifizierte Katalysatoren.

Gemäß WO 2012/126956 A1 wird die Hydrierung von Nitrilen bevorzugt bei einer Querschnittsbelastung der flüssigen Reaktionsphase im Bereich von 5 bis 50 $kg/m^2$*s durchgeführt. Diese hohen Querschnittsbelastungen werden in der Regel durch relativ hohe Umlaufströme (Rücklauf) im Verhältnis zum zugeführten Eduktstrom (Vorlauf) erzielt. So werden insbesondere Verhältnisse von Umlaufstrom zu zugeführtem Eduktstrom von 0,5:1 bis 250:1, und ganz

besonders bevorzugt von 2:1 bis 180:1 eingestellt. Die Reaktionsführung entspricht somit einem kontinuierlichen Kreislauf. Nachteilig dabei ist, dass eine entsprechende Verfahrensführung einen aufwändigen Reaktionsaufbau erfordert und durch die hohen Umlaufströme eine Rieselbettfahrweise der flüssigen und gasförmigen Edukte über das feste Katalysatorbett für die Erreichung eines hohen Umsatzgrades nicht durchgeführt werden kann.

Weiterhin sind Verfahren zur Herstellung von Isophorondiamin aus CN 104230721 B, WO 2012/076315 A1 und WO 2012/126869 A1 bekannt.

Gegenüber dem Stand der Technik stellt sich somit die Aufgabe, ein Verfahren zur Hydrierung von Nitrilverbindungen zu Aminoverbindungen bereitzustellen, das die Nachteile des Standes der Technik vermeidet. Insbesondere stellt sich die Aufgabe, ein Verfahren zur Hydrierung von Nitrilen zu Aminoverbindungen bereitzustellen, das technisch wenig aufwändig ist und die gewünschten Aminoverbindungen mit hohen Ausbeuten und Selektivitäten liefert.

Überraschenderweise wurde festgestellt, dass die vorliegende Aufgabe gelöst wird durch das erfindungsgemäße Verfahren zur Hydrierung von Nitrilverbindungen zu Aminoverbindungen, bei dem die Querschnittsbelastung des Reaktors bei der Hydrierung bezogen auf die Flüssigphase kleiner oder gleich 4,0 $kg/m^2*s$ beträgt

Unter Nitrilverbindungen sind hier und im Folgenden Nitrilgruppen (und ggf. weitere funktionelle Gruppen, wie z.B. Oxo-, Imino- oder Aminogruppen) aufweisende organische Verbindungen zu verstehen.

Bei dem erfindungsgemäßen Verfahren wird ein Gemisch, insbesondere ein Gemisch im Verhältnis von 50:50 bis 70:30, von 2,4,4-Trimethyl-Hexamethylendinitril und 2,2,4-Trimethyl-Hexamethylendinitril (TMN) zu einem Isomerengemisch bestehend aus 2,2,4-Trimethyl-Hexamethylendiamin und 2,2,4-Trimethyl-Hexamethylendiamin (TMD) oder Isophoronnitril oder Isophoronnitrilimin zu Isophorondiamin hydriert.

Ganz besonders geeignet, weil sich ein besonders einfacher Reaktoraufbau realisieren lässt, ist das erfindungsgemäße Verfahren zur aminierenden Hydrierung von Isophoronnitril zu Isophorondiamin geeignet, d.h. zur Umsetzung von Isophoronnitril mit Ammoniak und gleichzeitiger oder nachfolgender Hydrierung zum Isophorondiamin.

Bevorzugt wird dies ganz besonders bevorzugte Verfahren dabei so durchgeführt, dass

A) Isophoronnitril direkt in einer Stufe in Anwesenheit von Ammoniak, Wasserstoff, einem Katalysator und gegebenenfalls weiteren Zusätzen und in An- oder Abwesenheit von organischen Lösungsmitteln aminierend zu Isophorondiamin hydriert wird;
oder
B) Isophoronnitril in mindestens zwei Stufen umgesetzt wird, wobei dieses zunächst in einer ersten Stufe ganz oder teilweise in Isophoronnitrilimin umgewandelt wird, das als Reinsubstanz, oder im Gemisch mit anderen Komponenten und ggf. nicht umgesetzten Isophoronnitril, in mindestens einer nachfolgenden Stufe in Anwesenheit von mindestens Ammoniak, Wasserstoff und einem Katalysator zu Isophorondiamin hydriert wird.

Überraschend wurde gefunden, dass Querschnittsbelastungen des Reaktors bei der Hydrierung von kleiner oder gleich 4 $kg/m^2*s$ die sich stellenden erfindungsgemäßen Aufgaben lösen. Die Angabe der Querschnittsbelastung bezieht sich somit, insbesondere im Falle einer mehrstufigen aminierenden Hydrierung, nur auf den bzw. die Schritte, bei dem/denen Wasserstoff als Edukt zum Einsatz kommt.

Die Querschnittsbelastung des Reaktors berechnet sich anhand von Formel (1) über den Querschnitt A des Reaktors (Einheit [$m^2$]) und den Massendurchfluss m (Einheit: [kg/s]) an flüssigen bzw. gelösten Edukten, Ammoniak und gegebenenfalls Lösungsmitteln und/oder weiteren flüssigen Reaktionskomponenten (z.B. Rückflüssen). Die Gasphase der Reaktion (z.B. Wasserstoff, Inertgase) wird bei der Berechnung der Querschnittsbelastung nicht berücksichtigt.

$$Querschnittsbelastung = \frac{\dot{m}}{A} \left[\frac{kg}{m^2 * s}\right] \qquad (1)$$

Besonders gute Ergebnisse, insbesondere für die Synthese von Isophorondiamin aus Isophoronnitril oder Isophoronnitrilimin und für die Synthese von TMD aus 2,4,4-Trimethyl-Hexamethylendinitril und 2,2,4-Trimethyl-Hexamethylendinitril, können erzielt werden, wenn die Querschnittsbelastung 0,01 bis 4,0 $kg/m^2*s$, weiter bevorzugt 0,05 bis 3,0 $kg/m^2*s$ und ganz besonders bevorzugt 0,05 bis 2,0 $kg/m^2*s$ beträgt.

Bevorzugt wird das erfindungsgemäße Verfahren in einem Rohrreaktor durchgeführt. Bevorzugt handelt es sich um einen Festbettreaktor. Ganz besonders bevorzugt ist der Reaktor ein Rieselbettreaktor.

Der Reaktor wird erfindungsgemäß in einer kontinuierlichen Fahrweise betrieben, d.h. bei dem erfindungsgemäßen Verfahren handelt es sich um ein kontinuierliches Verfahren. Die flüssigen bzw. gelösten Edukte, ggf. erforderlicher Ammoniak und gegebenenfalls Lösungsmittel und/oder weitere flüssige Komponenten durchlaufen den Reaktor nur einmal ohne direkte Rückführung von flüssigen Reaktionsbestandteilen. Es ist allerdings möglich, dass unvollständig reagierte Bestandteile der Reaktion, ggf. anwesender Ammoniak und/oder Lösungsmittel nach Abtrennung dem Eduktstrom vor dem Eintritt in den Reaktor zugeführt werden.

Besonders bevorzugt, insbesondere bei der Hydrierung von Isophoronnitril oder Isophoronnitrilimin, liegt das Verhältnis an Umlaufstrom zum zugeführten Eduktstrom im Bereich von 0:1 bis 0,49:1, weiter bevorzugt im Bereich von 0:1 bis 0,25:1, ganz besonders bevorzugt im Bereich von 0:1 bis 0,1:1.

Die Hydrierung, insbesondere die aminierende Hydrierung von Isophoronnitril oder Isophoronnitrilimin, erfolgt bevorzugt bei Temperaturen zwischen 20 und 150°C, besonders bevorzugt 40 und 130°C, und Drücken von 0,3 bis 50 MPa, bevorzugt 5 bis 30 MPa.

Die Hydrierung kann in An- oder Abwesenheit eines Lösemittels erfolgen. Bevorzugt erfolgt die Hydrierung in Anwesenheit eines Lösemittels. Als Lösemittel können dem Fachmann bekannte und unter den o.g. Bedingungen einsetzbare Lösemittel eingesetzt werden. Bevorzugt erfolgt die Hydrierung in organischen Lösemitteln und/oder in flüssigem Ammoniak.

Eine bevorzugte Ausführungsform des erfindungsgemäßen Verfahrens ist ein Verfahren zur Herstellung von Isophorondiamin in einem zwei- oder mehrstufigen Prozess: In der ersten Stufe wird zumindest ein Teil des eingesetzten IPN bei An- oder Abwesenheit von Iminierungskatalysator oder Lösungsmittel durch Reaktion mit Ammoniak in Isophoronnitrilimin umgewandelt. Der Umsatz von IPN zu IPNI beträgt bevorzugt nach der Iminierung größer 80%, besonders bevorzugt größer 90%, ganz besonders bevorzugt größer 95%.

In mindestens einer weiteren, bevorzugt in einer zweiten, Stufe wird das Reaktionsprodukt der ersten Stufe, so wie es anfällt oder nach einer Weiterbehandlung und/oder Zugabe weiteren Ammoniaks, in Anwesenheit von Ammoniak und Wasserstoff und in An- oder Abwesenheit eines organischen Lösungsmittel bei einer Temperatur von 20 bis 150°C, bevorzugt 40 bis 130°C, und einem Druck von 0,3 bis 50 MPa, bevorzugt 5 bis 30 MPa, in Gegenwart mindestens eines Hydrierkatalysators hydriert.

In einer weiteren bevorzugten Ausführungsform erfolgt die Umsetzung von IPN zu IPDA in drei voneinander getrennten Reaktionsräumen. In dem ersten Reaktionsraum erfolgt die Umsetzung von IPN zu Isophoronnitrilimin mit überschüssigem Ammoniak an Iminierungskatalysatoren bei Temperaturen zwischen 20 und 150°C und Drücken zwischen 5 und 30 MPa. Im zweiten Reaktionsraum werden die entstandenen Reaktionsprodukte mit Wasserstoff in Gegenwart von überschüssigem Ammoniak an Hydrierkatalysatoren bei Temperaturen zwischen 20 und 130°C und Drücken von 5 bis 30 MPa hydriert. Im dritten Reaktionsraum werden die entstandenen Reaktionsprodukte an den erfindungsgemäß einzusetzenden Katalysatoren bei Temperaturen zwischen 100 und 160°C und Drücken von 5 bis 30 MPa hydriert.

Um die Gleichgewichtseinstellung der Iminierungsreaktion zu beschleunigen, ist es bevorzugt, einen Iminierungskatalysator zu verwenden. Hierzu können die nach dem Stand der Technik bekannten Iminierungskatalysatoren verwendet werden. Geeignete Katalysatoren sind beispielsweise anorganische oder organische Ionenaustauscher (siehe EP 0 042 119 B1), geträgerte Heteropolysäuren (siehe DE 44 26 472 A1), acide Metalloxide, insbesondere Aluminiumoxid und Titandioxid (siehe EP 0 449 089 B1), Sulfonsäuregruppen enthaltende Organopolysiloxane (DE 19627265.3) und saure Zeolithe sowie Aktivkohle (EP 0 623 585 A1). Bei Verwendung eines Iminierungskatalysators liegt die Reaktionstemperatur zwischen 10 und 150°C, vorzugsweise zwischen 30 und 130°C und ganz besonders bevorzugt zwischen 40 und 100°C. Der Druck liegt zwischen dem Eigendruck der Mischung und 50 MPa. Bevorzugt wird die Iminierungsreaktion bei dem Druck durchgeführt, bei dem auch die anschließende Hydrierung durchgeführt wird.

Obwohl die Iminierung von Isophoronnitril mit flüssigem Ammoniak bevorzugt ohne Zugabe weiterer Lösungsmittel durchgeführt wird, kann auch in Anwesenheit zusätzlicher Lösungsmittel gearbeitet werden. Geeignet sind einwertige Alkohole mit 1 bis 4 C-Atomen, insbesondere Methanol sowie Ether, besonders THF, MTBE und Dioxan.

In der Iminierungsstufe werden pro Mol eingesetztem IPN zwischen 1 und 500 Mol, bevorzugt 5 und 200 Mol, besonders bevorzugt zwischen 5 und 100 Mol Ammoniak eingesetzt. Typische Katalysatorbelastungen liegen im Bereich von 0,01 bis 10 kg IPN je kg Katalysator und Stunde, bevorzugt 0,5 bis 10 und besonders bevorzugt 0,5 bis 5 kg IPN je kg Katalysator und Stunde.

Bei der Iminierung in Gegenwart eines Iminierungskatalysators kann der Katalysator in Form eines Suspensionskatalysators oder Festbettkatalysators vorliegen. Vorteilhaft ist die Verwendung von Festbettkatalysatoren. In einer besonders bevorzugten Ausführungsform werden IPN und Ammoniak kontinuierlich von unten nach oben durch ein mit Iminierungskatalysator gefülltes Reaktionsrohr geleitet.

Die Hydrierung, insbesondere die Hydrierung von Isophoronnitril oder Isophoronnitrilimin oder von 2,4,4-Trimethyl-Hexamethylendinitril und 2,2,4-Trimethyl-Hexamethylendinitril, erfolgt bevorzugt bei Temperaturen zwischen 20 und 150°C, besonders bevorzugt 40 und 130°C, und Drücken von 0,3 bis 50 MPa, bevorzugt 5 bis 30 MPa. Es ist auch möglich, die Hydrierung in Gegenwart von Lösemitteln, insbesondere in Gegenwart der bei einer vorhergehenden Iminierungsstufe anwesenden Lösungsmittel durchzuführen. Der wesentliche Vorteil bei Verwendung eines Lösungsmittels liegt darin, dass die Hydrierung bei niedrigeren Drücken zwischen 0,3 und 10 MPa durchgeführt werden kann.

Der für die Hydrierung erforderliche Wasserstoff kann dem Reaktor entweder im Überschuss, beispielsweise mit bis zu 10.000 Moläquivalenten, zugeführt werden, oder nur in einer solchen Menge, dass der durch Reaktion verbrauchte Wasserstoff sowie der Teil des Wasserstoffs, der eingelöst im Produktstrom den Reaktor verlässt, nachgeführt wird. Bei kontinuierlicher Fahrweise kann der Wasserstoff im Gleich- oder Gegenstrom zugeführt werden.

In einer bevorzugten Ausführungsform erfolgt die Hydrierung in flüssigem Ammoniak als Lösungsmittel. Pro

Mol zu hydrierender Nitrilverbindung, bevorzugt pro Mol Isophoronnitril, Isophoronnitrilimin, 2,4,4-Trimethyl-Hexamethylendinitril oder 2,2,4-Trimethyl-Hexamethylendinitril, werden zwischen 1 und 500 Mol, bevorzugt 5 und 200 Mol, besonders bevorzugt zwischen 5 und 100 Mol Ammoniak verwendet. Zweckmäßigerweise kann im Falle einer vorgelagerten Iminierung die Ammoniakmenge bei der Hydrierung verwendet werden, die bei der vorhergehenden Stufe eingestellt wurde. Der Ammoniakanteil kann aber auch vor der Hydrierung durch Zugabe von zusätzlichem Ammoniak auf den gewünschten Wert erhöht werden.

Als Katalysatoren für die Hydrierung können prinzipiell alle Katalysatoren eingesetzt werden, die die Hydrierung von Nitril- und/oder Imingruppen mit Wasserstoff katalysieren. Bevorzugt wird als Katalysator ein Festbettkatalysator eingesetzt. Ganz besonders bevorzugt wird der Festbettkatalysator bei dem erfindungsgemäßen Verfahren in Rieselbettfahrweise eingesetzt.

Besonders geeignete Katalysatoren sind Nickel-, Kupfer-, Eisen-, Palladium-, Rhodium-, Ruthenium- und Cobaltkatalysatoren, ganz besonders Ruthenium- und Cobaltkatalysatoren. Zur Erhöhung der Aktivität, Selektivität und/oder Standzeit können die Katalysatoren zusätzlich Dotiermetalle oder andere Modifizierungsmittel enthalten. Typische Dotiermetalle sind z.B. Mo, Fe, Ag, Cr, Ni, V, Ga, In, Bi, Ti, Zr und Mn sowie die seltenen Erden. Typische Modifizierungsmittel sind z.B. solche, mit denen die Säure-Base-Eigenschaften der Katalysatoren beeinflusst werden können, bevorzugt Alkali- und Erdalkalimetalle bzw. deren Verbindungen, bevorzugt Mg- und Ca-Verbindungen, sowie Phosphorsäure oder Schwefelsäure sowie deren Verbindungen.

Die Katalysatoren können in Form von Pulvern oder Formkörpern, wie z.B. Extrudaten oder gepressten Pulvern, eingesetzt werden. Es können Vollkontakte, Raney-Typ-Katalysatoren oder Trägerkatalysatoren zur Anwendung kommen. Bevorzugt sind Raney-Typ- und Trägerkatalysatoren. Geeignete Trägermaterialien sind z B. Siliciumdioxid, Aluminiumoxid, Alumosilikate, Titandioxid, Zirkoniumdioxid, Kieselgur, Aluminium-Silicium-Mischoxide, Magnesiumoxid und Aktivkohle. Das Aktivmetall kann in dem Fachmann bekannter Weise auf das Trägermaterial aufgebracht werden, wie z.B. durch Imprägnierung, Aufsprühen oder Fällung. Je nach Art der Katalysatorherstellung sind weitere, dem Fachmann bekannte, Präparationsschritte notwendig, wie z.B. Trocknung, Calcinierung, Formgebung und Aktivierung. Zur Formgebung können optional weitere Hilfsstoffe wie z.B. Graphit oder Magnesiumstearat zugesetzt werden.

Es ist bevorzugt, dass die einzusetzenden Hydrierkatalysatoren vor ihrem Einsatz in der Hydrierung zunächst mit Ammoniak konditioniert werden. Dazu werden die Katalysatoren mit Ammoniak oder mit Mischungen aus Ammoniak und einem oder mehreren Lösungsmitteln in Kontakt gebracht. Bevorzugt erfolgt die Konditionierung nach Einbau der Katalysatoren im Hydrierreaktor, sie kann aber auch vor Einbau der Katalysatoren erfolgen. Zur Konditionierung werden zwischen 0,2 und 3, bevorzugt 0,5 und 2 $m^3$ Ammoniak pro $m^3$ Katalysator und Stunde eingesetzt. Üblicherweise wird bei Temperaturen zwischen 20 und 150°C, bevorzugt 40 bis 130°C, gearbeitet. Besonders bevorzugt wird eine Temperaturrampe durchfahren, bei der der Katalysator beginnend bei mäßig erhöhter Temperatur, bevorzugt zwischen 20 und 50°C, langsam bis auf die später für die Hydrierung gewünschte Reaktionstemperatur, bevorzugt 20 bis 150°C, aufgeheizt wird. Die Konditionierung wird bevorzugt in Gegenwart von Wasserstoff durchgeführt, wobei der Partialdruck des verwendeten Wasserstoffs im Reaktor den Bereich von 0,1 bis 50 MPa, bevorzugt 5 bis 40 MPa, besonders bevorzugt 10 bis 30 MPa umfasst. Die Zeitspanne der Konditionierung ist abhängig von der verwendeten Ammoniakmenge und liegt bevorzugt zwischen 1 und 48 h, besonders bevorzugt zwischen 12 und 24 h.

Bevorzugt wird für die Hydrierung ein Raney-Typ-Katalysator eingesetzt. Ein besonders bevorzugter Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:

| | |
|---|---|
| Cobalt: | 55 bis 95 Gew.-% |
| Aluminium: | 5 bis 45 Gew.-% |
| Chrom: | 0 bis 3 Gew.-% |
| Nickel: | 0 bis 7 Gew.-% |

Dieser bevorzugte Katalysator liegt bevorzugt in Form von unregelmäßigen Partikeln als Granulat vor und weist nach der Aktivierung Partikelgrößen von 1 bis 8 Millimeter (mm) auf.

Der bevorzugte Katalysator besteht aus einer Metall-Legierung, wobei die Metall-Legierung durch Basen auf der Oberfläche aktiviert ist. Die Schichtdicke der aktivierten Schicht auf der Partikeloberfläche des Katalysators beträgt bevorzugt 50 bis 1.000 Mikrometer ($\mu$m). Sie kann aber auch größer oder kleiner sein. Auf der Oberfläche befindet sich demnach die katalytisch aktive Zusammensetzung des Katalysators. Es ist aber auch im Rahmen der Erfindung möglich, das gesamte Katalysator-Partikel fast vollständig oder vollständig auszulaugen.

Die angegebenen Partikelgrößen können auch innerhalb des Bereichs eine statistische Verteilung der Größe aufweisen. Dabei sind enge Verteilungen als auch breite Verteilungen erfindungsgemäß.

Die Bestimmung der Partikelgrößen wird in der DIN ISO 9276-1 (September 2004) und 9276-2 (Februar 2006) und 9276-4 (Februar 2006) und 9276-6 (Januar 2012) beschrieben. Außerdem findet man genaue Angaben zur Definition

von Partikelgrößen, der Verteilung von Partikelgrößen und der Messung von Partikelgrößen in HORIBA® Scientific, A GUIDEBOOK TO PARTICLE SIZE ANALYSIS, 2012, von HORIBA® Instruments, Inc, Irvine, USA.

Erfindungsgemäß kann die Verteilung der Partikelgrößen und die Messung der Partikelgrößen durch Laserverfahren (ISO 13320, 2012), Lichtverfahren oder Bildverfahren bestimmt werden.

Bevorzugt wird der erfindungsgemäße Katalysator durch Siebung der hergestellten Granulate erhalten. Dabei werden sogenannte Siebfraktionen hergestellt. Dabei können einzelne Siebfraktionen gemischt werden, oder es wird ein Katalysator durch einmalige oder mehrmalige Siebung erhalten. Die so hergestellten Katalysatoren weisen bei den Partikelgrößen eine statistische Verteilung auf, üblicherweise in Form einer Gauß-Verteilung. Es sind symmetrische aber auch asymmetrische Verteilungen möglich.

Geeignete Methoden und Beschreibungen zur Siebanalyse sind beschrieben in:

DIN 66165-1:1987-04 Partikelgrößenanalyse; Siebanalyse; Grundlagen, und in DIN 66165-2:1987-04 Partikelgrößenanalyse; Siebanalyse; Durchführung.

Paul Schmidt, Rolf Körber, Matthias Coppers: Sieben und Siebmaschinen: Grundlagen und Anwendung. Wiley-VCH Verlag, 2003, ISBN 9783527302079, Kapitel 4.4: Analysesiebung. Jörg Hoffmann: Handbuch der Messtechnik. Hanser Verlag, 2007, ISBN 978-3-446-40750-3, Kapitel 3.12.16.2.1.

Allgemeine Methode zur Herstellung des Katalysators für die Hydrierung:

a) Herstellung der Legierung

Die Herstellung der Legierung erfolgt thermisch, zum Beispiel in einem Induktionsofen. Es werden dabei die Metalle geschmolzen und eine Legierung erhalten. Die fertige Schmelze wird für die Weiterverarbeitung zum Beispiel zu Barren gegossen.

b) Herstellung der Granulate

Die Legierung wird in geeigneten Geräten zu Granulaten verarbeitet, zum Beispiel über einen Backenbrecher vorzerkleinert und über eine Walzenmühle weiter gemahlen. Durch einen Siebschritt wird die gewünschte Größenverteilung der Granulate durch die Wahl der entsprechenden Siebe erhalten (z.B. 3-7 mm).

c) Aktivierung des Katalysators

Die Aktivierung des Katalysators erfolgt in geeigneten Apparaturen. Es können dabei organische oder anorganische Basen eingesetzt werden. Bevorzugt wird eine Lauge (z.B. Natronlauge), verwendet, wobei durch einen exothermen Vorgang ein Teil des Aluminiums unter Bildung von Wasserstoff und Aluminatlauge aus der Legierung herausgelöst wird. Die Konzentration der Lauge kann zwischen 5 und 30 Gew.-% liegen und die Reaktionstemperatur zwischen 50 und 100°C. Der Grad der Aktivierung wird über die Temperatur und die Reaktionszeit bestimmt. Dabei ist die Reaktionszeit variabel und ist abhängig von den Reaktionsbedingungen und dem gewünschten Grad der Aktivierung. Nach der Aktivierung wird der Katalysator mit Wasser gewaschen und anschließend unter Wasser gelagert.

Andere Zusammensetzungen können im Herstellungsschritt a) durch die entsprechende Wahl der Metallmengen analog produziert werden.

Bevorzugt wird der Katalysator in der beschriebenen Reihenfolge hergestellt. Die Aktivierung des Katalysators kann aber auch vor der Herstellung der Granulate erfolgen.

Zur Erhöhung der Aktivität, Selektivität und/oder Standzeit können die Katalysatoren zusätzlich Dotiermetalle oder andere Modifizierungsmittel enthalten. Typische Dotiermetalle sind z.B. Mo, Fe, Ag, V, Ga, In, Bi, Ti, Zr und Mn sowie die seltenen Erden, allein oder in Mischungen. Typische Modifizierungsmittel sind z. B. solche, mit denen die Säure-Base-Eigenschaften der Katalysatoren beeinflusst werden können, bevorzugt Alkali- und Erdalkalimetalle bzw. deren Verbindungen, bevorzugt Mg- und Li-Verbindungen. Für den Fall, dass derartige Verbindungen enthalten sind, in einer Menge von maximal 5 Gew.-%, reduziert sich der Anteil der oben genannten Metall Co und Al sowie gegebenenfalls Cr und Ni im Katalysator entsprechend, wobei die Anteile an Co und Al sowie gegebenenfalls Cr und Ni dann nach der Aktivierung sich zu mindestens 95 Gew.-% addieren, bezogen auf die enthaltenden Metalle.

**Beispiele**

**Herstellung des Katalysators, Co-Granulat:**

a) Herstellung der Legierung

Die Herstellung der Legierung erfolgt in einem Induktionsofen. Es werden dabei die Metalle in den entsprechenden Mengen bei 1.500°C geschmolzen. Die fertige Schmelze wird für die Weiterverarbeitung zu Barren gegossen.

b) Herstellung der Granulate

Die Legierungsbarren werden über einen Backenbrecher vorzerkleinert und über eine Walzenmühle weiter gemahlen. Durch einen Siebschritt wird die gewünschte Größenverteilung der Granulate durch die Wahl der entsprechenden Siebe erhalten.

c) Aktivierung des Katalysators

Die Aktivierung des Katalysators kann in einer Standard-Glaslaborapparatur erfolgen, z.B. in einem Becherglas. Zu den Granulaten wurde unter Rühren eine wässrige Lauge (z.B. Natronlauge) gegeben. Die Granulate befinden sich während der Aktivierung in einem Katalysatorkorb. Durch einen exothermen Vorgang wird ein Teil des Aluminiums unter Bildung von Wasserstoff und Natriumaluminatlauge aus der Legierung herausgelöst. Die Konzentration der eingesetzten Lauge lag bei 20 Gew.-% und die Reaktionstemperatur bei 90°C. Der Grad der Aktivierung wurde über die Reaktionszeit bestimmt. Nach der Aktivierung wird der Katalysator mit Wasser gewaschen und anschließend unter Wasser gelagert.

Der eingesetzte Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:

| | |
|---|---|
| Cobalt: | 55 Gew.-% |
| Aluminium: | 42 Gew.-% |
| Chrom: | 1 Gew.-% |
| Nickel: | 2 Gew.-% |

Es wurde eine Siebfraktion eingesetzt mit Partikelgrößen des Katalysators, also die Granulat-Teilchen mit einer statistischen Verteilung zwischen 2,0 bis 5,0 Millimeter (mm), wobei bis zu 10 Prozent der Partikel auch außerhalb des genannten Bereichs der genannten Untergrenze oder Obergrenze, aber auch jeweils bis zu 10 Prozent außerhalb des genannten Bereichs der genannten Untergrenze und Obergrenze, liegen können.

**Herstellung von IPDA**

Katalysatoren für die Herstellung von 3-Aminomethyl-3,5,5-Trimethylcyclohexylamin (Isophorondiamin, IPDA) aus 3-Cyano-3,5,5-Trimethylcyclohexanon (Isophoronnitril, IPN) werden in einem zweistufigen Verfahren auf ihr katalytische Wirksamkeit getestet.

In der ersten Reaktionsstufe wurde Isophoronnitril in Anwesenheit eines Iminierungskatalysators bei 45°C mit Ammoniak zumindest teilweise zu 3-Cyano-3,5,5-Trimethylcyclohexanimin überführt und in der zweiten Reaktionsstufe an einem Hydrierungskatalysator bei einer Temperatur von 100°C und einem Druck von 250 bar mit Wasserstoff unter Anwesenheit von Ammoniak aminierend hydriert. Jede Stufe der Herstellung wurde in einem separaten Reaktor mit individueller Temperierung durchgeführt. Beide Reaktoren waren dabei jedoch in Reihe geschaltet.

Der verwendete Hydrierreaktor hat einen Innendurchmesser von 2 cm und wurde mit 37 ml des zu prüfenden Katalysators befüllt. Die Einsatzlösung aus IPN (14,6 Gew.-%) und Ammoniak (85,4 Gew.-%) wurde mit einem Volumenstrom von 108 ml/h von oben nach unten durch das Reaktionsrohr gepumpt, was einer Querschnittsbelastung von 0,06 $kg/m^2*s$ entspricht. Der Wasserstoff wurde separat mit einem Volumenstrom von 40 Nl/h ebenfalls von oben zugegeben. Die Produktlösung wurde unter dem Reaktor in einem Abscheidegefäß aufgefangen und mittels Gaschromatographie auf ihre Zusammensetzung untersucht. Das Ergebnis in Tabelle 1 aufgeführt.

Tabelle 1:

| Temperatur | Querschnittsbelastung | Ausbeute IPDA / GC-% | Umsatz |
|---|---|---|---|
| 100°C | 0,06 $kg/m^2*s$ | 96,9 % | 99,9 % |

**Herstellung von TMD**

Katalysatoren für die Herstellung von Trimethyl-Hexamethylendiamin (TMD) aus Trimethyl-Hexamethylendinitril (TMN) werden in einem einstufigen, kontinuierlichen Verfahren auf ihre katalytische Wirksamkeit getestet.

In der Reaktion wurde TMN an einem Hydrierkatalysator (Cobalt-Granulat) bei einer Temperatur von 80°C und einem Druck von 250 bar mit Wasserstoff unter Anwesenheit von Ammoniak als Lösungsmittel hydriert.

Der verwendete Hydrierreaktor hat einen Innendurchmesser von 2 cm und wurde mit 42 ml des zu prüfenden Katalysators befüllt. Die Einsatzlösung aus TMN (14,6 Gew.-%) und Ammoniak (85,4 Gew.-%) wurde mit einem Volumenstrom von 120 ml/h von oben nach unten durch das Reaktionsrohr gepumpt, was einer Querschnittsbelastung von 0,08 $kg/m^2*s$ entspricht. Der Wasserstoff wurde separat mit einem Volumenstrom von 40 Nl/h ebenfalls von oben zugegeben. Die Produktlösung wurde unter dem Reaktor in einem Abscheidegefäß aufgefangen und mittels Gaschromatographie auf ihre Zusammensetzung untersucht. Das Ergebnis in Tabelle 2 aufgeführt.

Tabelle 2:

| Temperatur | Querschnittsbelastung | Ausbeute TMD / GC-% | Umsatz |
|---|---|---|---|
| 80°C | 0,08 $kg/m^2*s$ | 91,3 % | 99,9 % |

**Patentansprüche**

1. Verfahren zur Hydrierung von Nitrilverbindungen ausgewählt aus

   - Gemischen von 2,4,4-Trimethyl-Hexamethylendinitril und 2,2,4-Trimethyl-Hexamethylendinitril,
   - Isophoronnitril und
   - Isophoronnitrilimin

   zu Aminoverbindungen, **dadurch gekennzeichnet, dass** die Querschnittsbelastung des Reaktors bei der Hydrierung bezogen auf die Flüssigphase kleiner oder gleich 4,0 $kg/m^2*s$ beträgt.

2. Verfahren nach Anspruch 1 , **dadurch gekennzeichnet, dass** Isophoronnitril aminierend zu Isophorondiamin hydriert wird.

3. Verfahren nach Anspruch 2, **dadurch gekennzeichnet, dass**

   A) Isophoronnitril direkt in einer Stufe in Anwesenheit von Ammoniak, Wasserstoff, einem Katalysator und gegebenenfalls weiteren Zusätzen und in An- oder Abwesenheit von organischen Lösungsmitteln aminierend zu losphorondiamin hydriert wird;
   oder
   B) Isophoronnitril in mindestens zwei Stufen umgesetzt wird, wobei dieses zunächst in einer ersten Stufe ganz oder teilweise in Isophoronnitrilimin umgewandelt wird, das als Reinsubstanz, oder im Gemisch mit anderen Komponenten und ggf. nicht umgesetzten Isophoronnitril, in mindestens einer nachfolgenden Stufe in Anwesenheit von mindestens Ammoniak, Wasserstoff und einem Katalysator zu Isophorondiamin hydriert wird.

4. Verfahren nach einem der vorhergehenden Ansprüche, **dadurch gekennzeichnet, dass** die Querschnittsbelastung 0,01 bis 4,0 $kg/m^2*s$, weiter bevorzugt 0,05 bis 3,0 $kg/m^2*s$ und ganz besonders bevorzugt 0,05 bis 2,0 $kg/m^2*s$ beträgt.

5. Verfahren nach einem der vorhergehenden Ansprüche, **dadurch gekennzeichnet, dass** das Verhältnis an Umlaufstrom zum zugeführten Eduktstrom im Bereich von 0:1 bis 0,49:1 liegt.

6. Verfahren nach einem der vorhergehenden Ansprüche, **dadurch gekennzeichnet, dass** die Hydrierung bei Temperaturen zwischen 20 und 150°C und Drücken von 0,3 bis 50 MPa durchgeführt wird.

7. Verfahren nach Anspruch 2 oder 3, **dadurch gekennzeichnet, dass** das Verfahren zur Herstellung von Isophorondiamin zwei- oder mehrstufig durchgeführt wird und dass in einer ersten Stufe Isophoronnitril bei An- oder Abwesenheit von Iminierungskatalysator oder Lösungsmittel durch Reaktion mit Ammoniak in Isophoronnitrilimin umgewandelt wird.

**8.** Verfahren nach Anspruch 7, **dadurch gekennzeichnet, dass** das Reaktionsprodukt der ersten Stufe in Anwesenheit von Ammoniak und Wasserstoff und in An- oder Abwesenheit eines organischen Lösungsmittels bei einer Temperatur von 20 bis 150°C und einem Druck von 0,3 bis 50 MPa in Gegenwart mindestens eines Hydrierkatalysators hydriert wird.

**9.** Verfahren nach einem der vorhergehenden Ansprüche, **dadurch gekennzeichnet, dass** für die Hydrierung ein Katalysator ausgewählt aus der Gruppe bestehend aus Nickel-, Kupfer-, Eisen, Palladium-, Rhodium-, Ruthenium- und Cobaltkatalysatoren eingesetzt wird.

**10.** Verfahren nach Anspruch 9, **dadurch gekennzeichnet, dass** der Hydrierkatalysator ausgewählt aus der Gruppe bestehend aus Raney-Typ- und Trägerkatalysatoren ist.

**11.** Verfahren nach Anspruch 10, **dadurch gekennzeichnet, dass** der Katalysator nach der Aktivierung in seiner Gesamtheit eine Zusammensetzung aufweist in Gewichtsprozent bezogen auf alle Anteile der enthaltenden Metalle:

| | |
|---|---|
| Cobalt: | 55 bis 95 Gew.-% |
| Aluminium: | 5 bis 45 Gew.-% |
| Chrom: | 0 bis 3 Gew.-% |
| Nickel: | 0 bis 7 Gew.-% |

**Claims**

**1.** Process for hydrogenating nitrile compounds selected from

- mixtures of 2,4,4-trimethylhexamethylenedinitrile and 2,2,4-trimethylhexamethylenedinitrile,
- isophoronenitrile and
- isophoronenitrile imine

to amino compounds, **characterized in that** the cross-sectional loading of the reactor during the hydrogenation is less than or equal to 4.0 $kg/m^2*s$, based on the liquid phase.

**2.** Process according to Claim 1, **characterized in that** isophoronenitrile is subjected to aminating hydrogenation to give isophoronediamine.

**3.** Process according to Claim 2, **characterized in that**

A) isophoronenitrile is subjected directly in one stage to aminating hydrogenation to give isophoronediamine in the presence of ammonia, hydrogen, a catalyst and possibly further additions, and in the presence or absence of organic solvents;
or
B) isophoronenitrile is reacted in at least two stages, wherein said isophoronenitrile is initially converted in a first stage entirely or partly to isophoronenitrile imine which, as a pure substance or in a mixture with other components and possibly unreacted isophoronenitrile, is hydrogenated in at least one subsequent stage to give isophoronediamine in the presence of at least ammonia, hydrogen and a catalyst.

**4.** Process according to any of the preceding claims, **characterized in that** the cross-sectional loading is 0.01 to 4.0 $kg/m^2*s$, more preferably 0.05 to 3.0 $kg/m^2*s$ and especially preferably 0.05 to 2.0 $kg/m^2*s$.

**5.** Process according to any of the preceding claims, **characterized in that** the ratio of circulation stream to the feedstock stream supplied is in the range from 0:1 to 0.49:1.

**6.** Process according to any of the preceding claims, **characterized in that** the hydrogenation is carried out at temperatures between 20 and 150°C and pressures of 0.3 to 50 MPa.

**7.** Process according to Claim 2 or 3, **characterized in that** the process for preparing isophoronediamine is carried

out in two or more stages and **in that** isophoronenitrile is converted in a first stage to isophoronenitrile imine by reaction with ammonia in the presence or absence of an imination catalyst or solvent.

**8.** Process according to Claim 7, **characterized in that** the reaction product of the first stage is hydrogenated in the presence of at least one hydrogenation catalyst in the presence of ammonia and hydrogen and in the presence or absence of an organic solvent at a temperature of 20 to 150°C and a pressure of 0.3 to 50 MPa.

**9.** Process according to any of the preceding claims, **characterized in that** a catalyst is used for the hydrogenation selected from the group consisting of nickel, copper, iron, palladium, rhodium, ruthenium and cobalt catalysts.

**10.** Process according to Claim 9, **characterized in that** the hydrogenation catalyst is selected from the group consisting of Raney-type and supported catalysts.

**11.** Process according to Claim 10, **characterized in that** the catalyst after activation has a composition in its entirety, in percent by weight based on all proportions of metals present, of

| | |
|---|---|
| cobalt: | 55 to 95 wt% |
| aluminium: | 5 to 45 wt% |
| chromium: | 0 to 3 wt% |
| nickel: | 0 to 7 wt%. |

## Revendications

**1.** Procédé d'hydrogénation de composés de nitrile choisis parmi :

- les mélanges de 2,4,4-triméthyl-hexaméthylènedinitrile et de 2,2,4-triméthyl-hexaméthylènedinitrile,
- l'isophorone-nitrile et
- l'isophorone-nitrilimine,

en composés amino, **caractérisé en ce que** le chargement de section transversale du réacteur lors de l'hydrogénation par rapport à la phase liquide est inférieur ou égal à 4,0 kg/m$^2$*s.

**2.** Procédé selon la revendication 1, **caractérisé en ce que** de l'isophorone-nitrile est soumis à une hydrogénation aminante pour former de l'isophoronediamine.

**3.** Procédé selon la revendication 2, **caractérisé en ce que**

A) de l'isophorone-nitrile est soumis à une hydrogénation aminante directement en une étape en la présence d'ammoniac, d'hydrogène, d'un catalyseur et éventuellement d'autres additifs et en la présence ou en l'absence de solvants organiques, pour former de l'isophorone-diamine ;
ou
B) de l'isophorone-nitrile est mis en réaction en au moins deux étapes, celui-ci étant tout d'abord transformé lors d'une première étape en totalité ou en partie en isophorone-nitrilimine, qui est hydrogénée sous la forme d'une substance pure, ou en mélange avec d'autres composants et éventuellement de l'isophoronenitrile non réagi, lors d'au moins une étape ultérieure en la présence d'au moins de l'ammoniac, de l'hydrogène et un catalyseur, pour former de l'isophorone-diamine.

**4.** Procédé selon l'une quelconque des revendications précédentes, **caractérisé en ce que** le chargement de section transversale est de 0,01 à 4,0 kg/m$^2$*s, de manière davantage préférée de 0,05 à 3,0 kg/m$^2$*s et de manière tout particulièrement préférée de 0,05 à 2,0 kg/m$^2$*s.

**5.** Procédé selon l'une quelconque des revendications précédentes, **caractérisé en ce que** le rapport entre le courant de circulation et le courant de réactifs introduit se situe dans la plage allant de 0:1 à 0,49:1.

**6.** Procédé selon l'une quelconque des revendications précédentes, **caractérisé en ce que** l'hydrogénation est réalisée

à des températures comprises entre 20 et 150 °C et des pressions de 0,3 à 50 MPa.

**7.** Procédé selon la revendication 2 ou 3, **caractérisé en ce que** le procédé de fabrication d'isophoronediamine est réalisé en deux ou davantage d'étapes, et **en ce que**, lors d'une première étape, de l'isophoronenitrile est transformé en isophorone-nitrilimine en la présence ou en l'absence d'un catalyseur d'imination ou d'un solvant par réaction avec de l'ammoniac.

**8.** Procédé selon la revendication 7, **caractérisé en ce que** le produit de réaction de la première étape est hydrogéné en la présence d'ammoniac et d'hydrogène et en la présence ou en l'absence d'un solvant organique à une température de 20 à 150 °C et une pression de 0,3 à 50 MPa, en la présence d'au moins un catalyseur d'hydrogénation.

**9.** Procédé selon l'une quelconque des revendications précédentes, **caractérisé en ce qu'**un catalyseur choisi dans le groupe constitué par les catalyseurs à base de nickel, de cuivre, de fer, de palladium, de rhodium, de ruthénium et de cobalt est utilisé pour l'hydrogénation.

**10.** Procédé selon la revendication 9, **caractérisé en ce que** le catalyseur d'hydrogénation est choisi dans le groupe constitué par les catalyseurs de type Raney et supportés.

**11.** Procédé selon la revendication 10, **caractérisé en ce que** le catalyseur présente après l'activation dans sa totalité une composition en pourcentage en poids par rapport à toutes les proportions des métaux contenus :

| | |
|---|---|
| cobalt : | 55 à 95 % en poids, |
| aluminium: | 5 à 45 % en poids, |
| chrome : | 0 à 3 % en poids, |
| nickel : | 0 à 7 % en poids. |

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 3352913 A
- EP 0042119 B1
- EP 0449089 B1
- EP 0816323 A1
- DE 4426472 A1
- EP 0623585 A1
- EP 0394967 A1
- EP 0913387 B1
- WO 2012126956 A1
- CN 104230721 B
- WO 2012076315 A1
- WO 2012126869 A1
- DE 19627265


### In der Beschreibung aufgeführte Nicht-Patentliteratur


- **PAUL SCHMIDT ; ROLF KÖRBER ; MATTHIAS COPPERS.** Sieben und Siebmaschinen: Grundlagen und Anwendung. Wiley-VCH Verlag, 2003
- **JÖRG HOFFMANN.** Handbuch der Messtechnik. Hanser Verlag, 2007